# EUROPEAN PATENT APPLICATION

(11) **EP 2 282 207 A1**
(43) Date of publication of application: **09.02.2011**
(21) Application number: 10189652.0
(22) Date of filing: 04.06.2004
(51) Int. Cl.: G01N 33/545, B05D 7/24, C12N 5/00

(54) **PLASMA POLYMERISATION METHODS FOR THE DEPOSITION OF CHEMICAL GRADIENTS AND SURFACES DISPLAYING A GRADIENT OF IMMOBILISED BIOMOLECULES**

(30) Priority: 12.06.2003 GB 0313569
(62) Divisional of application: 04736065.6
(71) Applicant: Becton, Dickinson and Company, Franklin Lakes, New Jersey 07417-1880 (US)
(72) Inventor: The designation of the inventor has not yet been filed
(74) Representative: Harrison Goddard Foote

(57) **Abstract**

The invention relates to a method to prepare at least part of at least one surface of a substrate comprising depositing on the surface at least one plasma monomer from a monomer source wherein during deposition of said monomer said monomer and/or said surface are moved relative to one another to provide a non-uniform plasma polymerised surface, and introducing to at least part of said plasma polymerised surface a binding entity to provide a non-uniform surface formed from said binding entity.

## Description

The invention relates to a method to manufacture a non-uniform binding surface, products comprising a surface obtainable by said method and methods of separation, screening and cell culture using said surface.

Plasma polymerisation is a technique which allows an ultra-thin (eg ca.200nm) cross linked polymeric film to be deposited on substrates of complex geometry and with controllable chemical functionality. As a consequence, the surface chemistry of materials can be modified, without affecting the bulk properties of the substrate so treated. Plasmas or ionised gases are commonly excited by means of an electric field. They are highly reactive chemical environments comprising ions, electrons, neutrals (radicals, metastables, ground and excited state species) and electromagnetic radiation. At reduced pressure, a regime may be achieved where the temperature of the electrons differs substantially from that of the ions and neutrals. Such plasmas are referred to as "cold" or "non-equilibrium" plasmas. In such an environment many volatile organic compounds (eg volatile alcohol containing compounds, volatile acid containing compounds, volatile amine containing compounds, or volatile hydrocarbons, neat or with other gases, eg Ar, have been shown to polymerise (H.K. Yasuda, Plasma Polymerisation, Academic Press, London 1985) coating both surfaces in contact with the plasma and those downstream of the discharge. The organic compound is often referred to as the "monomer". The deposit is often referred to as "plasma polymer".

The advantages of such a mode of polymerisation potentially include: ultra-thin pinhole free film deposition; plasma polymers can be deposited onto a wide range of substrates; the process is solvent free and the plasma polymer is free of contamination. Under conditions of low power, typically 10⁻² W/cm³, plasma polymer films can be prepared which retain a substantial degree of the chemistry of the original monomer. For example, plasma polymerised films of acrylic acid contain the carboxyl group (Haddow et al., Langmuir, Vol 16: 5654-60, 2000). The low power regime may be achieved either by lowering the continuous wave power, or by pulsing the power on and off.

Co-polymerisation of one or more compounds having functional groups with a hydrocarbon allows a degree of control over surface functional group concentrations in the resultant plasma copolymer (PCP) (Beck et al., Polymer 37: 5537-5539, 1996). Suitably, the monomers are ethylenically unsaturated. Thus the functional group compound maybe unsaturated carboxylic acid, alcohol or amine, for example, whilst the hydrocarbon is suitably an alkene. By plasma polymerisation, it is also possible to deposit ethylene oxide-type molecules (eg. tetraethyleneglycol monoallyl ether) to form 'non-fouling' surfaces (Beyer et al., Journal of Biomedical Materials Research 36: 181-9, 1997). It is also possible to deposit perfluoro-compounds (i.e. perfluorohexane, hexafluoropropylene oxide) to form hydrophobic/superhydrophobic surfaces (Coulson et al., Chemistry of Materials 12: 2031-2038, 2000).

This technique is advantageous because the surfaces have unique chemical and physical characteristics. For example, the surfaces have increased affinity for biological molecules exposed to said surface and allow the assaying of the bound molecule. The surfaces are uniform and enable the reproducible and sensitive assaying of biological molecules bound to the surface. Similarly, the surface wettability, adhesion and frictional/wear characteristics of the substrate can be modified in a controllable and predictable manner.

The method disclosed herein allows the provision of surfaces that are non-uniform and define local surface regions that have different chemical and/or physical properties. We refer to these surfaces as "patterned" in both chemistry and topography. The effect is achieved by drawing off a proportion of the plasma through a micrometre scale orifice(s) which is translated across the surfaces to be patterned. Alternatively, a plasma may be excited at the tip, or within a microcapilliary which can then be used to "write" the molecular architecture and chemistry onto the surface. Chemistry and molecular architecture maybe varied vertically (Z-direction) and/or laterally (X-Y plane) by changing the key plasma parameters (power, flow rate, pulse duty cycle or monomer composition), or by altering the portion of the plasma 'drawn off' by physical, electrical or magnetic means during writing. These surfaces allow the immobilisation of different molecules and concentrations of molecules at a micron scale. Similarly, this technique may be used to control the local wettability, adhesion and frictional/wear characteristics on a surface, and have application in microfluidics.

In WO01/31339 we disclose the treatment of products by plasma polymerisation

The technique disclosed in WO01/31339, although effective with respect to providing uniform plasma polymerised surfaces to which biomolecules bind with specificity and affinity, is not sufficiently versatile to provide a surface which has diverse chemical or physical properties.

We herein disclose a method we refer to as "plasma writing" which provides surfaces that are characterised by chemical and structural micropatterns or gradients extending, typically into three dimensions, wherein the X-Y plane is defined by the surface, and the Z-direction is substantially perpendicular thereto. The method creates both chemical and molecular architectures on a surface, to give rise to two or three-dimensional patterns, without the need to prefabricate masks or stencils, as described in Dai et al., Journal of Physical Chemistry B 101:9548-54 (1997) and without limitation in the number or type of different architectures created on a single surface as part of the same process.

There is a requirement to provide non-uniform plasma polymer surfaces which have been adapted to provide complex heterochemical surfaces to which biological entities can differentially bind.

According to a first aspect of the invention there is provided a method to prepare at least part of at least one surface of a substrate comprising
i.) depositing on the surface at least one plasma monomer from a monomer source wherein during deposition of said monomer said monomer and/or said surface are moved relative to one another to provide a non-uniform plasma polymerised surface; and
ii) introducing to at least part of said plasma polymerised surface a binding entity to provide a non-uniform surface formed from said binding entity.

Non-uniform refers to surfaces which have a heterogeneous chemical and/or physical structure.

As used herein, a "binding entity" means any entity which interacts covalently and/or non-covalently with functional groups of the plasma polymerised surface or in turn to binding entities which have been immobilised on the plasma polymerised surface.

Binding entities may include cells, metabolites, pharmaceutically active agents, proteins e.g cells, hormones, antibodies, enzyme, receptor; macromolecules, e.g DNA, RNA, protein fragments, peptides, polypeptides; ligands, proteoglycans, carbohydrates, nucleotides, oligonucleotides, toxic reagents, chemical species. The binding entities may together form one or more binding surfaces.

The binding entity may provide a surface onto which at least one cell can grow or attach. For example, the binding entity may comprise a chemical functional group such as a carboxyl or amine functional group (R.M.France, R.D.Short, R.A.Dawson, S.MacNeil. Journal of Materials Chemistry, 1998, Vol.8, p37-42). Alternatively, the binding entity may comprise an immobilised or adsorbed biological entity (protein, protein fragment, peptide sequence, etc.) see C.R.Jenney, J.M.Anderson. Journal of Biomedical Materials Research, 2000, Vol.50, p281-290.

Typically, the binding entity is immobilised on the plasma polymer surface. The binding entity may be chemically linked to functional groups in the plasma polymer surface.

In a preferred embodiment, the invention provides a method to prepare at least part of at least one surface of a substrate comprising
i.) depositing on the surface at least one plasma monomer from a monomer source wherein during deposition of said monomer said monomer and/or said surface are moved relative to one another to provide a non-uniform plasma polymerised surface;
ii.) introducing to at least part of said plasma polymerised surface a first binding entity to provide a non-uniform surface formed from said binding entity; and
iii.) contacting said first binding entity with a second binding entity which binds said first binding entity.

In an alternative aspect, step (i) of the method comprises
i.) depositing on the surface at least one plasma monomer from at least two spatially separated monomer sources to provide a non-uniform plasma polymerised surface.

The non-uniform plasma polymerised surface may be prepared by placing a substrate in the region of diffusive mixing between two spatially separated monomer sources and exciting a plasma in this region. The deposited material will have a chemistry/chemical composition that correlates with the mix of monomers in the region of mixing.

The invention herein disclosed enables the "overwriting" of deposited plasma polymers having different chemistries and molecular architecture in a spatially restricted pattern (optionally at varying concentration and at a micrometer resolution) with other entities e.g biological molecules. This in turn allows the production of complex heterochemical surfaces with highly defined chemical and physical surface properties.

The invention advantageously facilitates the binding and/or separation of different biological molecules or agents and different concentrations of biological molecules/agents followed by their detection and analysis; locally modifies the surface characteristics such as wettability, friction and wear, and adhesion. The invention also facilitates the culturing and screening of cells.

In a preferred method of the invention there is provided a surface comprising two or more plasma polymers formed from at least two monomers, preferably a plurality of plasma polymers formed from a plurality of monomers.

In a further preferred method of the invention said surface comprises at least one plasma polymer of at least one monomer wherein the concentration of said plasma polymer is non-uniform across said surface, or part thereof.

In a further preferred method of the invention, said surface comprises of two or more plasma polymers of two or more monomers, wherein the concentration of at least one plasma polymer is non-uniform across said surface, or part thereof.

In a further preferred method of the invention said monomer is a volatile alcohol.

In an alternative method of the invention said monomer pattern is a volatile acid.

In a still further alternative method said monomer is a volatile amine.

In a further method of the invention said monomer is a volatile hydrocarbon.

In a yet further preferred method of the invention said monomer is a volatile fluorocarbon.

In a still further preferred method of the invention said monomer is an ethyleneoxide-type molecule.

In a further preferred method of the invention said monomer is a volatile siloxane.

In yet still a further preferred method of the invention said monomer is at least one of selected from the group consisting of N-vinyl pyrolidone, allyl alcohol; acrylic acid; octa-1,7-diene; allyl amine; perfluorohexane; tetraethyleneglycol monoallyl ether; or hexamethyl disiloxane (HMDSO).

In a further preferred method of the invention said polymer consists of a single monomer.

Preferably the monomer consists essentially of an ethylenically unsaturated organic compound.

Preferably the monomer consists of essentially of a single ethylenically unsaturated organic compound.

Preferably the monomer consists of an ethylene oxide type molecule. (e.g. Triglyme)

Preferably the compound is an alkene (eg containing up to 20 carbon atoms and more usually up to 12 carbon atoms, eg 8), a carboxylic acid (especially α,β - unsaturated carboxylic acid, for example acrylic or methacrylic acid); an alcohol (especially an unsaturated alcohol); or an amine (especially an unsaturated amine).

Preferably the monomer consists of a mixture of two or more ethylenically unsaturated organic compounds.

Preferably the compounds are selected from the group consisting of: an alkene (eg containing up to 20 carbon atoms and more usually up to 12 carbon atoms, eg 8), a carboxylic acid (especially α,β - unsaturated carboxylic acid); an alcohol (especially an unsaturated alcohol); or an amine (especially an unsaturated amine).

"Alkene" refers to linear and branched alkenes, of which linear are preferred, containing one or more than one C=C double bond eg an octadiene such as octa-1,7-diene. Dienes form a preferred class of alkenes.

The monomer may consist essentially of a saturated organic compound. The monomer may consist of an aromatic compound, a heterocyclic compound or a compound containing one or more carbon-carbon triple bonds.

Alternatively said polymer is a co-polymer. Preferably said co-polymer comprises at least one organic monomer with at least one hydrocarbon. Preferably said hydrocarbon is an alkene, eg a diene such as, for example octa 1,7-diene.

The method also encompasses the use of other compounds to form plasma, for example and not by way of limitation, ethylamine; heptylamine; methacrylic acid; propanol, hexane, acetylene or diaminopropane.

Preferably the monomer is a polymerisable monomer having a vapour pressure of at least 6.6x10⁻² mbar. Monomers with a vapour pressure of less than 1.3x10⁻² mbar are generally not suitable unless their vapour pressure can be raised sufficiently by heating.

In a preferred method of the invention said monomer (s) is/are deposited on said surface in spatially separated dots.

In a further preferred method of the invention said monomer (s) is/are deposited on said surface in tracks or lines.

In a yet further preferred method of the invention, said dots and/or lines may be of different polymer chemistry.

In a still further preferred method of the invention, the chemical composition and/or functionality of the line, track or dot may be non-uniform along its length and in height.

In a yet further preferred method of the invention, the line or track may be in the form of loops or closed circuits.

In a yet further preferred method of the invention regions which do not consist of a deposited plasma polymer may be comprised of polymerised ethylene-oxide type monomer providing a non-binding surface.

In a preferred method of the invention said plasma is sustained under low power conditions, from which are obtainable films containing the original monomer chemistry. Typically, low power conditions refer to a continuous wave power of 10⁻² W/cm³, or the equivalent time-averaged power in the case of pulsed plasmas.

According to a further aspect of the invention there is provided a substrate comprising a surface obtainable by the method according to the invention.

In a yet further aspect, the invention provides a substrate having a plasma polymerised surface wherein said surface has at least first and second polymer areas wherein the composition of said first area is different from said second area characterised in that at least one area has bound thereto a first binding entity to which a second binding entity is bound.

Preferably said substrate is selected from the group consisting of: glass; plastics (e.g. polyethylene terephthalate, high density polyethylene, low density polyethylene, polyvinyl chloride, polypropylene or polystyrene); nitrocellulose, Poly (vinylidene fluoride) (PVdF), polycarbonate, poly (methylmathacrylate) or nylon, metal, ceramics, quartz, composite structures (e.g. metal film on glass) or silicon wafer.

The invention encompasses a plasma polymerised surface that has along at least one axis (XYZ), typically along its length, a variable concentration of plasma or polymer which is herein referred to as a "plasma gradient surface". Further, the invention encompasses surfaces comprising multiple plasma polymers deposited in a controlled manner.

The invention thus provides a substrate comprising a plasma gradient surface to which binding entities are introduced thereby providing a non-uniform binding surface or "gradient binding surface".

The invention encompasses a substrate comprising a combinatorial gradient binding surface wherein one or more binding entities are introduced to provide multidirectional gradients of functional groups provided on the substrate surface, possibly along varying axis (XYZ). The invention thus provides a binding surface containing a spatially varying concentration of binding entity which is directly related to the original concentration of functional groups on the surface.

In one embodiment, the invention provides a substrate having a binding surface comprising a spatially varying concentration of functional groups. The concentration of functional groups may vary along the same axis of the surface.

In an alternative embodiment, the invention provides a substrate having a binding surface comprising a spatially varying concentration of proteins. The concentration of proteins/ligands may vary along the same axis of the surface, for example, a surface may have along one axis an increasing concentration of protein (A) and, in the same direction along the same or other axis, a decreasing concentration of ligand (B).

Gradients may be gradients of concentration, charge, pH, hydrophobicity, size etc.

According to a further aspect there is provided a product comprising a substrate according to the invention.

In a preferred embodiment of the invention said product is part of an assay product.

In a further preferred embodiment of the invention said assay product is a microarray.

In an alternative preferred embodiment said assay product is microtitre plate.

In an alternative preferred embodiment said product comprises a microfluidic device, or a part thereof (e.g. valve, switch, guide channel, binding site, pump).

According to a yet further aspect of the invention there is provided an assay product according to the invention for use with an array printer.

According to a further aspect of the invention there is provided an assay product according to the invention for use with an array reader.

In a further aspect the invention provides a cell culture system comprising a substrate according to the invention wherein the binding entity provides a surface onto which at least one cell can grow.

In a yet further aspect the invention provides a cell culture system comprising a substrate comprising a surface obtainable by depositing on at least part of at least one surface of said substrate a non-uniform plasma polymer surface.

In a preferred embodiment of the invention said culture system is part of an assay product.

In a further preferred embodiment of the invention said assay product is a microarray.

In an alternative preferred embodiment said assay product is microtitre plate.

According to a yet further aspect of the invention there is provided an assay product according to the invention for use with an array printer.

According to a further aspect of the invention there is provided an assay product according to the invention for use with an array reader.

In a further aspect the present invention provides a method of separating biological molecules comprising using a substrate according to the invention to separate biological molecules on the basis of differential binding studies.

The substrate could be used to separate mixtures of biomolecules on the basis of difference in physical or chemical properties. (for example, mass, charge, size, hydrophobicity, pH). Moreover, the substrate could be used to separate out identical mixtures by different properties (charge, size, pH etc.).

As used herein, "biomolecules" includes, but is not limited to, cells, metabolites, pharmaceutically active agents, proteins e.g hormones, antibodies, enzyme, receptor; macromolecules, e.g DNA, RNA, proteins, peptides, polypeptides; ligands, proteoglycans, carbohydrates, nucleotides, oligonucleotides, toxic reagents, chemical species.

In a preferred embodiment, the invention provides a method of separating cells on a substrate according to the invention.

The invention enables the migration of cells to different positions on the surface of the substrate to be observed which may give an indication of the cells' viability. Cell motility may be an indicator of disease states, and hence it may be possible to diagnose these states from the behaviour of populations of cells. It has been observed that the motility of cancerous cells is different to that of non-cancerous cells *in vivo,* therefore, the invention may be useful in the diagnosis of cancer.

In a further aspect the invention provides a method of screening comprising using a substrate according to the invention to determine the differential binding of biomolecules to said substrate. The method may be useful in the identification of potential therapeutic agents by binding studies.

In a preferred embodiment the invention provides a method of screening comprising:
i) preparing a substrate according to the invention;
ii.) screening the surface of said substrate to determine the binding property of a biomolecule to said surface, wherein said binding property is identifiable by its binding position on said surface;
iii.) identification of biomolecules with said binding property.

As used herein, the "binding property" of a biomolecule, for example a cell, refers to the attachment or release characteristics of the molecule, for example, the rate of attachment or release of a molecule to/from the surface, viability of cells with respect to differences in surface concentration of the binding entity, attachment and proliferation of cells on the surface with respect to differences in surface concentration of the binding entity, changes in the expressed protein, or ability to react to secondary stimuli for cells with respect to differences in surface concentration of the binding entity, adsorption (physical or chemical) of biological molecules (proteins, ligands, peptides, nucleic acids, glycoproteins, etc.) with respect to differences in surface concentration of the binding entity, changes in conformation of adsorbed biological molecules with respect to differences in surface concentration of the binding entity.

The present invention therefore provides a method and products for rapidly investigating and/or screening a multiplicity of binding entities. The method may comprise evaluating and optionally selecting a binding surface composition by differential binding studies of biomolecules to said surface. The invention thus makes possible binding surfaces having a variety of biological applications e.g cell culture, adsorption of proteins, cell or biomolecule separation, studies on protein to protein or cell to cell interactions.

The method further provides a screen for determining the effect of the interaction between the binding surface of the substrate and the biomolecule e.g cell on its behaviour e.g. viability.

It will be apparent that the invention relates to the provision of plasma polymerised surfaces that are physically non-uniform and which we refer to as "patterned". The invention relates to the provision of surfaces of more than one single patterned chemistry (indeed, there is no practical limit on the number) that can be 'drawn' with micrometre precision. Such surfaces cannot be obtained by the stencil approach of Dai et al. (Journal of Physical Chemistry B 101: 9548-5 4, 1997). The morphology of the substrate merely affects the maximum resolution of the plasma pattern.

Patterns may consist of lines, circles, loops, arrays, or any conceivable geometric shape in any combination on a scale from centimetres down to around 5 microns. This includes 3-dimensional patterns where the material along the z-axis (height) also exhibits chemical and physical differences. As such, nanometer features may be 'grown' on surfaces which comprise different strata of "chemistry" on different local regions of the surface.

A polymer may be deposited from virtually any compound (particularly organic compounds), provided it can be induced to form a plasma. Typically this means that the compound must be volatile, although this may be done by heating or by use of a carrier gas, for example monomers having a vapour pressure of at least 6.6x10⁻² mbar at room temperature. Hence a microdot or array thereof, or a microtrack may be produced which contains any chemical functional group and there is no limit to the number of different chemistries that may be deposited onto a single substrate. This is in contrast to previously disclosed methods of patterning plasma polymers, which are only capable of depositing 'monotone' patterns. The written polymer does not necessarily contain any functional groups at all - a hydrocarbon starting compound will deposit an essentially functionally blank surface. The provision of functionalised patterns on a non-fouling surface can be achieved by writing on a surface which has first been uniformly plasma polymerised by an ethylene oxide type monomer. Currently disclosed methods for patterning of plasma polymers do not allow the production of surfaces containing more than one chemistry. In addition, using this method of writing polymer onto a substrate permits formation on surface of closed loops and circuits - an aspect of surface patterning precluded by use of an overlayed mask.

To form a gradient of chemistry, the composition of the plasma is changed concomitantly with the relative movement of the writing element with respect to the surface. Such a change in the composition of the plasma may be achieved by changing the temperature of the monomer(s), increasing the partial pressure or mixing ratio of the monomer(s) or carrier gas(es), or by changing the amplitude, or pulse regime, or frequency of the power input into the system.

The substrate and plasma source are affixed to either side of a precision XYZ translation stage. The XYZ stage comprises one fixed and one travelling flange. Therefore, the substrate and plasma source are moved relative to each other.

Preferred features of each aspect of the invention are as for each of the other aspects *mutates mutandis.*

The invention will now be described by examples only and with reference to the following figures, materials and methods;
Figure 1 is a plasma polymerisation apparatus;
Figure 2 is a graph showing the elemental composition, determined by XPS, of a gradient of acrylic acid/allylamine over a distance of 11mm.
Figure 3 is a graph showing the COOR concentration of a gradient surface as a function of position along it as analysed by XPS

### Materials and Methods

The methodology of plasma polymerisation is disclosed in WO01/31339 and is incorporated by reference in its entirety.

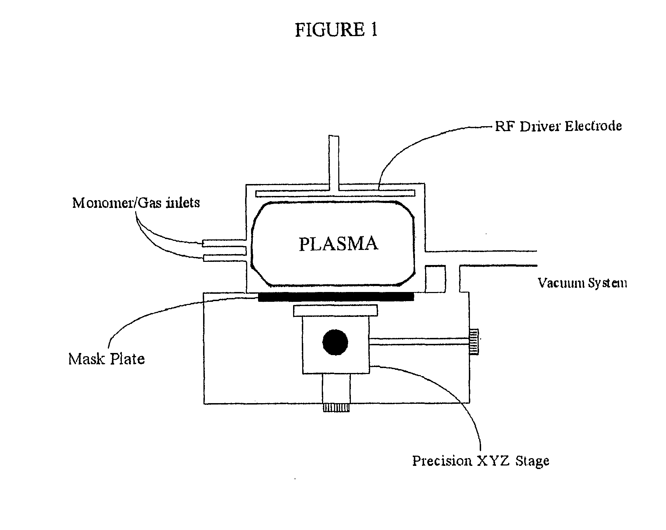

The schematic diagram of the plasma "writing" apparatus is shown in figure 1.

The apparatus consists of two vacuum chambers separated by a Mask Plate, but sharing a common vacuum system. The topmost chamber has several monomer input ports and an electrode for exciting a plasma. The lower chamber contains a precision XYZ manipulation stage, upon which is mounted the substrate to be patterned.

The 'writer' element consists of a 'nib' which contains a small feature which is used to 'write' chemistry onto the surface. Examples of such nibs include single holes, multiple holes, and single or multiple slots where the dimensions may range from 2 microns up to several centimetres, but more typically lie in the range 5-1000 microns. The nib may be an integral part of the plasma source in (for example) the case of a microcapilliary. In this case the term 'nib' refers to the aperture at the end of the capilliary.

A plasma is initiated of such a composition (of monomer or monomers, or monomer(s) in conjunction with carriers gas or gases) as would be required to deposit a uniform film of the desired composition as described in WO01/31339. Typically a monomer consists of an organic compound which may be induced to exist in the gas phase either by heating, or spraying, or by the use of a carrier gas, or by its own vapour pressure at room temperature or below. Pressure within the plasma chamber is typically around 1x10⁻² mbar, and normally within the range 10⁻³ mbar - 1 mbar. Working pressures for plasma polymerisation are normally between 10⁻⁵ mbar and atmospheric pressure, or higher.

Other plasma systems, for example, microwave, pulsed rf, dc, , atmospheric, microdischarge, microcapillary, may be used and the means of adapting the above description to allow these plasma sources to be integrated will be clear to one skilled in the art.

The writing element is translated across the surface of a sample mounted on an XYZ manipulator. Either the sample, or the plasma source, or both may be moved relative to the other. Such movement may be controlled manually, or by the action of computer controlled motors to describe the desired feature shape onto the surface. The rate of movement may be easily calculated by knowing the dimensions of the writing element, the deposition rate of the plasma polymer, and the required thickness of the deposited film.

To form a gradient of chemistry, the composition of the plasma is changed concomitantly with the relative movement of the writing element with respect to the surface. Such a change in the composition of the plasma may be achieved by changing the temperature of the monomer(s), increasing the partial pressure or mixing ratio of the monomer(s) or carrier gas(es), or by changing the amplitude, or pulse regime, or frequency of the power input into the system. Other means of altering the plasma composition are known in the art.

The sample is raised so as to be extremely close to the Mask Plate (but without touching). The mask plate consists of a stainless steel plate, with a small aperture that defines the features to be deposited. The nature of the deposition is such that the plasma is guided by the aperture and forms a polymeric deposit on the surface beneath it. Note however, that this aperture is used almost as a 'pen' to write functionalised polymeric material onto the substrate, as opposed to a simple 'stencil' to form an image on the surface.

Both chambers are evacuated using a common vacuum system consisting of a turbomolecular pump backed by a two-stage rotary pump. The base pressure of the whole apparatus is ~ 10⁻⁵ mbar.

A plasma is excited in the top chamber, by means of an rf generator (Coaxial Power Systems, UK), and by adjusting the flow rate of the monomer/monomers and the power and pulse regime of the plasma the desired plasma composition is selected.

### "Writing" of Plasma Gradients

A functionality gradient was deposited by using two different monomer compounds. Allylamine and acrylic acid were obtained from Aldrich (UK) and subjected to several freeze-pump-thaw cycles to remove dissolved gases. A mask consisting of a single -100 micron hole was attached to the mask plate, and a piece of silicon wafer as substrate was raised as close as possible to the mask without touching (as described above). Initially, a plasma was excited using only the acrylic acid monomer feed. The mixture of monomer gases was then varied concomitantly with the linear movement of the sample beneath the mask. Hence the initial deposition was comprised wholly of acrylic acid plasma polymer, while later deposition consisted of a mixture of allylamine and acrylic acid, and the final portion of the deposition consisted wholly of allylamine. Thus over the range of motion of the sample during the experiment, the surface composition changed smoothly from one dominated by carboxylic acid groups, to one in which amine groups dominated.

Microgradients are not simply limited to bi-functional gradients, any number of monomers could be used to produce continuously varying surface features. Similarly, gradients of other properties can be envisaged; gradients of wettability (from ultra-hydrophobic through to hydrophilic), gradients of crosslink density, adhesivity and variations of thickness. A gradient can be formed which comprises a chemically continuous region connecting any two or more polymers with different properties, irrespective of what those properties might be. A polymer can be seen as occupying a point in an n-dimensional parameter-space - there will always be a direct path between two such points, which is independent of the dimensionality of the parameter space.

The examples described above use a feature scale of around 100 microns, to illustrate the techniques. In practice it might be required that surfaces are patterned on a millimetre or centimetre scale as an upper boundary, right down to 1 micron at the bottom end.

There are variations that could be made to the plasma system to control the plasma writing process. Plasma may be excited using DC, radiofrequency (pulsed or continuous wave) or microwave radiation, or it may be excited within, or at the tip of a micrometre scale capillary. There may be carrier gases involved for some less-volatile monomers. The processes may benefit from a simple computer system to manage the plasma parameters and position of the XYZ stage for improved accuracy and automation of the writing process. Further, although the experiments described the plasma as being in a top chamber, and the sample in a lower chamber, the pattern formation requires only that the sample be isolated from the plasma by the mask plate, irrespective of orientation of the components of the system.

It is possible to change the plasma composition in the region of the mask by means of applied electric and magnetic fields. These might be used as 'lenses' to further focus the plasma. They may also be used to increase or decrease the relative contributions of the ionic and radical components of the plasma- in extremis reducing the species arriving at the substrate to a collimated beam of radical species, or low energy beam of ions.

In addition to directly depositing onto the substrate material, pretreatments may be employed to clean the surface, or to etch topographic features into the substrate prior to writing. This allows the construction of 3-dimensional functionalised structures on surfaces (for example a 'trench' with amine functional groups deposited along the bottom) in a single process.

Microgradients could be used to separate mixtures of biomolecules on the basis of difference in physical or chemical properties. (for example, mass, charge, size, hydrophobicity). This is analogous to gel electrophoresis, and gel permeation chromatography. Gradients could be used to separate out identical mixtures by different properties (charge, size, etc.).

The chemistry of the written features may range from non-functional hydrocarbon surfaces (deposited from alkane, alkene, aromatic type compounds) to any other conceivable chemical group. For example, amines, acids, alcohols, ethers, esters, imines, amides, keytones, aldehydes, anhydrides, halogens, thiols, carbonyls, silicones, fluorocarbons. Additionally, plasma polymers which are electrically conducting may be deposited. The only limitation on the functionality incorporated is that there must exist a starting compound that is capable of being induced to exist in the gas phase (with or without heating) at low pressure (above ∼10⁻⁵ mbar). Different chemistries may also be formed using reactive (N₂, O₂, H₂O), or non-reactive (Ar) gases. These gases may also be used to etch features into the substrate - all as part of the same process.

Patterns may be produced that contain a mixture of any number of the above functionalities in any combination or arrangement on the same substrate material. Surfaces that contain gradients of functionality on a scale of centimetres, down to around 10 microns are possible. A gradient is a region of continuous change between two different chemistries. A gradient can always be constructed between any two regions of different chemistry, in the same way that a straight line can always be drawn through two points in space.

The polymer micropatterns, microarrays, microgradients and microtracks may be written onto any substrate material. For example, glasses, ceramics, metals, semiconductors, and polymers including (but not limited to) polycarbonate (PC), polystyrene (PS), polyethyleneterephthalate (PET), polymethylmethacrylate (PMMA), polyvinylchloride (PVC), polytetrafluoroethylene (PTFE).

### EXAMPLES

### Preparation of plasma polymer surfaces

### Example 1

Acrylic acid and allylamine monomers were obtained from Aldrich (UK) and used as received, save for several freeze-pump-thaw cycles to remove dissolved gases prior to use. A 13 mm glass coverslip was used as a substrate material and was attached to the XYZ sample stage and pumped down to the system base pressure (<10⁻³mbar). A 1cm writing element was used and was initially placed so that the whole sample surface was exposed to the plasma. A plasma consisting of 4cm³ ₛₜₚmin⁻¹ of allylamine was excited in the plasma chamber at a continuous wave power of 5W and a reactor pressure of 1.9x10⁻² mbar. The writing element was moved across the surface at a rate of 1mm/min for a period of 13 minutes. Simultaneously, the flow rate of allylamine was reduced by slowly adjusting the needle valve, and replaced by a flow of acrylic acid vapour such that after 12 minutes, the monomer flow consisted of only acrylic acid at a flowrate of 4cm³ₛₜₚmin⁻¹ and a pressure of ∼2x10⁻² mbar. At all times the total monomer flow rate was maintained at 4 cm3ₛₜₚmin-1. (The ratio of the two monomer flow rates in cm³ ₛₜₚmin⁻¹ is equivalent to their molar ratio assuming ideal behaviour).

In order to analyse the gradual change of chemistry along the sample surface, it was analysed using X-Ray photoelectron spectroscopy at 500 micron intervals across the cover slip. The elemental composition at each point is shown in Figure 4 as a ratio of oxygen/carbon and nitrogen/carbon.

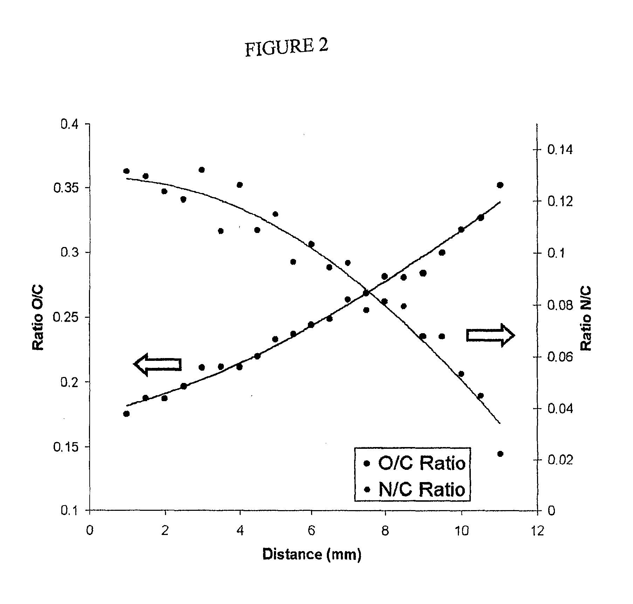

Figure 2 shows a gradient of oxygen and nitrogen chemistry which was changed concomitantly with the motion of the writing element from a composition of 100% allylamine to 100% acrylic acid at a constant power of 5W and a movement rate of the sample relative to the writing element of 1.0mm/min.

### Attachment of biotin to a gradient of amine groups

### Example 2

A gradient is produced using the method described above, but with allylamine and 1,7-octadiene as monomers. The analysis of the gradient will show that the surface concentration of amine groups decreases across the length of the gradient. N-Hydroxysuccinimidobiotin (NHS-Biotin) (Sigma, Dorset) is dissolved to 25mM in dimethylsulfoxide and then further diluted to 1mM with distilled water. The gradient is incubated for 24h at room temperature in the dark with 1ml of this NHS-Biotin solution. The surface is then washed thoroughly with distilled water to remove any non-reacted NHS-Biotin.

The density of biotin across the surface is measured by incubating (at 37 degrees C) the gradient with a solution of avidin-fluoresceinisothiocyanate (Avidin-FTTC) in phosphate buffered saline (PBS) at a concentration of 225 micrograms per mil for a period of 30 minutes. Avidin binds very strongly to the surface-bound biotin, and the gradient is then to be imaged using a fluorescence microscope.

The surface of cells are conjugated with biotin. An avidin solution is used to connect the two biotin molecules and link the cells to the surface.

### Culture of cells on a gradient of acid groups

### Example 3

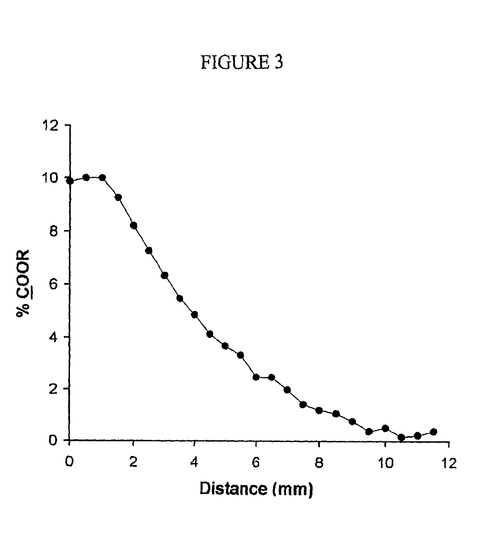

A gradient was produced using the method described above, but with acrylic acid and 1,7-octadiene as monomers. Analysis of the gradient (shown in Figure 3) indicates a falling concentration of acid functional groups along the length of the gradient. In two separate experiments, human fibroblasts and melanocytes were cultured on the gradient surfaces under standard culture conditions (with serum).

The invention further includes the subject matter of the claims of WO2004/111648 from which this application is derived, the content of which is reproduced below as numbered paragraphs.
Paragraph 1. A method to prepare at least part of at least one surface of a substrate comprising
   i) depositing on the surface at least one plasma monomer from a monomer source wherein during deposition of said monomer said monomer and/or said surface are moved relative to one another to provide a non-uniform plasma polymerised surface; and
   ii) introducing to at least part of said plasma polymerised surface a binding entity to provide a non-uniform surface formed from said binding entity.
Paragraph 2. A method as described in paragraph 1 wherein the binding entity interacts covalently with functional groups of the plasma polymerised surface.
Paragraph 3. A method as described in paragraph 1 wherein the binding entity interacts non-covalently with functional groups of the plasma polymerised surface.
Paragraph 4. A method as described in paragraph 2 or 3 wherein a further binding entity interacts with the binding entities which have been introduced on the plasma polymerised surface.
Paragraph 5. A method as described in paragraph 1 wherein the binding entity is selected from the group consisting of cells, metabolites, pharmaceutically active agents, proteins including hormones, antibodies, enzyme, receptor; macromolecules including DNA, RNA, protein fragments, peptides, polypeptides; ligands, proteoglycans, carbohydrates, nucleotides, oligonucleotides, toxic reagents and chemical species.
Paragraph 6. A method as described in paragraph 1 wherein the method comprises the steps of
   i.) depositing on the surface at least one plasma monomer from a monomer source wherein during deposition of said monomer said monomer and/or said surface are moved relative to one another to provide a non-uniform plasma polymerised surface;
   ii.) introducing to at least part of said plasma polymerised surface a first binding entity to provide a non-uniform surface formed from said binding entity; and
   iii.) contacting said first binding entity with a second binding entity which binds said first binding entity.
Paragraph 7. A method as described in paragraph 1 wherein the method comprises the steps of
   i) depositing on the surface at least one plasma monomer from at least two spatially separated monomer sources to provide a non-uniform plasma polymerised surface;
   ii.) introducing to at least part of said plasma polymerised surface a first binding entity to provide a non-uniform surface formed from said binding entity; and
   iii.) contacting said first binding entity with a second binding entity which binds said first binding entity.
Paragraph 8. A method as described in paragraph 1 wherein the monomer is a volatile alcohol.
Paragraph 9. A method as described in paragraph 1 wherein the monomer is a volatile acid.
Paragraph 10. A method as described in paragraph 1 wherein the monomer is a volatile amine.
Paragraph 11. A method as described in paragraph 1 wherein the monomer is a volatile hydrocarbon.
Paragraph 12. A method as described in paragraph 1 wherein the monomer is a volatile fluorocarbon.
Paragraph 13. A method as described in paragraph 1 wherein the monomer is an ethyleneoxide-type molecule.
Paragraph 14. A method as described in paragraph 1 wherein the monomer is a volatile siloxane.
Paragraph 15. A method as described in paragraph 1 wherein the monomer is selected from the group consisting of N-vinyl pyrolidone, allyl alcohol; acrylic acid; octa-1,7-diene; allyl amine; perfluorohexane; tetraethyleneglycol monoallyl ether and hexamethyl disiloxane (HMDSO).
Paragraph 16. A method as described in paragraph 1 wherein the polymer consists of a single monomer.
Paragraph 17. A method as described in paragraph 16 wherein the monomer consists essentially of an ethylenically unsaturated organic compound.
Paragraph 18. A method as described in paragraph 17 wherein the monomer consists essentially of a single ethylenically unsaturated organic compound.
Paragraph 19. A method as described in paragraph 18 wherein the monomer consists of an ethylene oxide type molecule.
Paragraph 20. A method as described in paragraph 17 wherein the monomer consists of a mixture of two or more ethylenically unsaturated organic compounds.
Paragraph 21. A method as described in paragraph 17 wherein the compound is selected from the group consisting of an alkene containing up to 20 carbon atoms, a carboxylic acid, an alcohol and an amine.
Paragraph 22. A method as described in paragraph 1 wherein the polymer is a co-polymer.
Paragraph 23. A method as described in paragraph 22 wherein the co-polymer comprises at least one organic monomer with at least one hydrocarbon.
Paragraph 24. A method as described in paragraph 1 wherein the monomer is a polymerisable monomer having a vapour pressure of at least 6.6x10⁻² mbar.
Paragraph 25. A method as described in paragraph 1 wherein the monomer (s) is/are deposited on said surface in spatially separated dots.
Paragraph 26. A method as described in paragraph 1 wherein the monomer (s) is/are deposited on said surface in tracks or lines.
Paragraph 27. A method as described in paragraph 25 or 26 wherein the chemical composition and/or functionality of the line, track or dot is non-uniform along its length.
Paragraph 28. A method as described in paragraph 1 wherein the chemical composition and/or functionality of the line, track or dot is non-uniform in its height.
Paragraph 29. A method as described in paragraph 1 wherein the surface comprises non-plasma deposited regions that are comprised of polymerised ethylene-oxide type monomer to provide a non-binding surface.
Paragraph 30. A substrate comprising a surface obtainable by the method according to paragraph 1.
Paragraph 31. A substrate comprising a plasma polymerised surface wherein said surface comprises at least first and second polymer areas and wherein the composition of said first area is different from said second area characterised in that at least one area has bound thereto a first binding entity to which a second binding entity is bound.
Paragraph 32. A substrate as described in paragraph 30 or 31 wherein the substrate is selected from the group consisting of glass, plastics, nitrocellulose, Poly vinylidene fluoride (PVdF), polycarbonate, poly (methylmathaerylate), nylon, metal, ceramics, quartz, composite structures and silicon wafer.
Paragraph 33. A substrate as described in paragraph 30 or 31 wherein the plastic is selected from the group consisting of polyethylene terephthalate, high density polyethylene, low density polyethylene, polyvinyl chloride, polypropylene and polystyrene.
Paragraph 34. A product comprising a substrate as described in paragraph 30 or 31.
Paragraph 35. A product as described in paragraph 34 wherein said product is part of an assay product.
Paragraph 36. A product as described in paragraph 35 wherein said assay product is a microarray.
Paragraph 37. A product as described in paragraph 35 wherein said assay product is a microtitre plate.
Paragraph 38. A product as described in paragraph 34 wherein said product comprises a microfluidic device or a part.
Paragraph 39. A cell culture system comprising a substrate as described in paragraph 30 or 31 wherein the binding entity provides a surface onto which at least one cell can grow.
Paragraph 40. A cell culture system as described in paragraph 39 wherein the system is part of an assay product.
Paragraph 41. Use of a substrate as described in paragraph 30 or 31 in the separation of biological molecules.
Paragraph 42. Use as described in paragraph 41 wherein the biological molecule is selected from the group consisting of cells, metabolites, pharmaceutically active agents, proteins including hormones, antibodies, enzyme, receptor; macromolecules, including DNA, RNA, proteins, peptides, polypeptides; ligands, proteoglycans, carbohydrates, nucleotides, oligonucleotides, toxic reagents and chemical species.
Paragraph 43. A method of screening biological molecules comprising the steps of
   i) preparing a substrate as described in paragraph 30 or 31;
   ii.) screening the surface of said substrate to determine the binding property of a biological molecule to said surface, wherein said binding property is identifiable by its binding position on said surface; and
   iii.) identifying the biological molecule with said binding property.

## Claims

1. A method to prepare at least part of at least one surface of a substrate, comprising:
i) depositing on a surface of a substrate at least one plasma monomer from a monomer source, wherein said surface is separated from said monomer source by a mask plate having an aperture and wherein during deposition of said monomer, said monomer and/or said surface are moved relative to one another to provide a non-uniform plasma polymerised surface with said aperture defining features of the deposited plasma polymerised surface, wherein said mask plate does not touch said surface.

2. A method according to claim 1, further comprising introducing to at least part of said plasma polymerised surface a binding entity to provide a non-uniform surface formed from said binding entity, wherein the binding entity provides a surface onto which a cell can grow or attach.

3. A method according to claim 2, wherein the binding entity comprises a carboxyl or amine functional group.

4. A method according to claim 2, wherein the binding entity is selected from the group consisting of cells, metabolites, pharmaceutically active agents, proteins, or protein fragments, including hormones, antibodies, enzyme, receptor; macromolecules including DNA, RNA, protein fragments, peptides, polypeptides; ligands, proteoglycans, carbohydrates, nucleotides, oligonucleotides, toxic reagents and chemical species or wherein the binding entity comprises an immobilised or adsorbed biological entity.

5. A method according to claim 2, wherein the binding entity interacts covalently with functional groups of the plasma polymerised surface.

6. A method according to claim 2, wherein the binding entity is immobilised on the plasma polymer surface.

7. A method according to claim 2, wherein the binding entity is chemically linked to functional groups in the plasma polymer surface.

8. A method according to claim 2, wherein the binding entity interacts non-covalently with functional groups of the plasma polymerised surface.

9. A method according to claim 2, wherein a cell interacts with the binding entity of the plasma polymerised surface.

10. A method according to claim 1, wherein the monomer is a volatile alcohol, volatile acid, volatile amine, volatile hydrocarbon, volatile fluorocarbon, ethyleneoxide-type molecule, or a volatile siloxane.

11. A method according to claim 1, wherein the monomer is selected from the group consisting of N-vinyl pyrolidone, allyl alcohol; acrylic acid; octa-1,7-diene; allyl amine; perfluorohexane; tetraethyleneglycol monoallyl ether and hexamethyl disiloxane (HMDSO).

12. A method according to claim 1, wherein the polymer consists of a single monomer.

13. A method according to claim 12, wherein the monomer consists essentially of an ethylenically unsaturated organic compound, a single ethylenically unsaturated organic compound, an ethylene oxide type molecule, or a mixture of two or more ethylenically unsaturated organic compounds.

14. A method according to claim 13, wherein the compound is selected from the group consisting of an alkene containing up to 20 carbon atoms, a carboxylic acid, an alcohol and an amine.

15. A method according to claim 1, wherein the polymer is a co-polymer.

16. A method according to claim 15, wherein the co-polymer comprises at least one organic monomer with at least one hydrocarbon.

17. A method according to claim 1, wherein the monomer is a polymerisable monomer having a vapour pressure of at least 6.6x10⁻² mbar.

18. A method according to claim 1, wherein the monomer (s) is/are deposited on said surface in spatially separated dots, or in tracks or lines.

19. A method according to claim 18, wherein the chemical composition and/or functionality of the line, track or dot is non-uniform along its length, or in its height.

20. A method according to claim 1, wherein the surface comprises non-plasma deposited regions that are comprised of polymerised ethylene-oxide type monomer to provide a non-binding surface.

21. A method according to any preceding claim, wherein the substrate is selected from the group consisting of glass, plastics, nitrocellulose, Poly vinylidene fluoride (PVdF), polycarbonate, poly (methylmathacrylate), nylon, metal, ceramics, quartz, composite structures and silicon wafer.

22. A method of screening biological molecules comprising the steps of
i) depositing on a surface of a substrate at least one plasma monomer from a monomer source, wherein said surface is separated from said monomer source by a mask plate having an aperture and wherein during deposition of said monomer, said monomer and/or said surface are moved relative to one another to provide a non-uniform plasma polymerised surface with said aperture defining features of the deposited plasma polymerised surface, wherein said mask plate does not touch the surface;
ii) screening the surface of said substrate to determine the binding property of a cell to said surface, wherein said binding property is identifiable by its binding position on said surface; and
iii) identifying the cell with said binding property.

23. A method according to claim 22, further comprising introducing to at least part of said plasma polymerised surface a binding entity to provide a non-uniform surface formed from said binding entity.

24. A method according to claim 23 or claim 24, wherein the method comprises the steps of
i) depositing on the surface at least one plasma monomer from at least two spatially separated monomer sources to provide a non-uniform plasma polymerised surface;
ii) introducing to at least part of said plasma polymerised surface a first binding entity to provide a non-uniform surface formed from said binding entity; and
iii) contacting said first binding entity with a second binding entity which binds said first binding entity.
